# EUROPEAN PATENT APPLICATION

(11) **EP 1 454 634 A1**
(43) Date of publication of application: **08.09.2004**
(21) Application number: 02803548.3
(22) Date of filing: 20.11.2002
(51) Int. Cl.: A61K 47/38, A61K 9/22, A61K 31/4439, A61K 47/04, A61K 47/10, A61K 47/14, A61K 47/32, A61K 47/44, A61P 1/04

(54) **PREPARATION COMPOSITIONS CONTAINING ACID-UNSTABLE PHYSIOLOGICALLY ACITVE COMPOUNDS AND PROCESS FOR PRODUCING THE SAME**

(30) Priority: 21.11.2001 JP 2001356018
(71) Applicant: Eisai Co., Ltd., Tokyo 112-8088 (JP)
(72) Inventor: AOKI, Shigeru, Kawashima-cho, Hashima-gun, Gifu 501-602 (JP)
(74) Representative: HOFFMANN EITLE
(86) International application number: PCT/JP2002/012132
(87) International publication number: WO 2003/043661

(57) **Abstract**

It is intended to disclose preparation composition which enable convenient production of sustained-release preparation composition containing acid-unstable physiologically active compound and have the desired sustained-release properties. Namely, preparation composition comprising a core containing an acid-unstable physiologically active compound and a coating with which the core is coated and which contains a mixture of a water-insoluble polymer and an enteric polymer. The preparation composition according to the present invention makes it possible to maintain an effective concentration in blood of the physiologically active compound over a long time, thereby improving its therapeutic effect. Moreover, the composition according to the present invention remains stable over prolonged storage.

## Description

### TECHNICAL FIELD

This invention relates to a sustained-release preparation composition, and more particularly relates to a preparation composition that continuously releases a gastric acid secretion inhibitor as an acid-unstable active ingredient, and that is very suited to biological use, and to a process for producing the composition.

### BACKGROUND ART

A benzimidazole-based compound used as a physiologically active compound, or an alkali metal salt, alkaline earth metal salt, or other physiologically acceptable salt thereof, is a peptic ulcer therapeutic drug that has a powerful inhibitory action on what are known as proton pumps, and that suppresses gastric acid very well. It is well-known that examples of such therapeutic drugs at present include omeprazole, lansoprazole, rabeprazole, and pantoprazole.

The action of the above-mentioned compounds is continuous and more powerful than that of histamine H₂ receptor antagonists, and these drugs are currently administered just once a day. However, there have been early clinical indications that if these drugs are taken in the morning, it may be difficult to suppress the secretion of gastric acid during sleep.

Accordingly, it is predicted that therapeutic efficacy would be improved by imparting the desired sustained release property to a therapeutic agent containing the above-mentioned benzimidazole-based compound, and thereby maintaining the effective concentration of this agent in the blood.

Nevertheless, it is difficult to sustain release enough to maintain the blood concentration with a preparation composition in which the above-mentioned physiologically active compound (or a preparation composition containing this physiologically active compound) is covered with just an enteric polymer. Also, with a preparation composition in which the above-mentioned physiologically active compound (or a preparation composition containing this physiologically active compound) is covered with just a water-insoluble polymer, the elution of the physiologically active compound so slow that there is the possibility that this active compound will be decomposed in the gastric acid. Furthermore, if the coverage amount of water-insoluble polymer is increased in order to suppress decomposition in gastric acid, the elution of the active compound slows down to the point that a sufficient therapeutic efficacy is not obtained.

It has been proposed that a benzimidazole-based compound (the physiologically active compound) can be released gradually if a matrix is formed with a higher alcohol or a fatty acid ester (see Patent Document 1), but it is still possible that the physiologically active compound will be decomposed by gastric acid. It has also been proposed that a sustained-release preparation composition can be obtained by providing an elution suppressing film on the inside of an enteric coating of an enteric preparation containing omeprazole (see Patent Document 2).

However, it is known empirically that further covering the coating of a preparation already covered with the elution suppressing film can damage the elution suppressing film, thereby affecting the function thereof and also changing the elution behavior of the physiologically active compound in the preparation, so considerable care must be taken in the coating step (operation) in which the elution suppressing film is covered. With a process based on this technology (see Patent Document 3), after the elution suppressing film has been applied, it is necessary to further cover the enteric coating on the film, so coating solutions for the film and the coating have to be prepared separately, which is the problem in that it complicates production.

The present invention was conceived in light of these problems, and it is a particular object thereof to provide a preparation composition that makes possible the simple production of a sustained-release preparation composition containing an acid-unstable benzimidazole-based compound, that has the desired sustained release, that maintains its effective blood concentration over an extended period of time and thereby improves therapeutic efficacy, and that is stable during extended storage, and to provide a process for producing the composition.
Patent Document 1: WO 00/074654
Patent Document 2: WO 99/32091
Patent Document 3: WO 98/32091

### DISCLOSURE OF INVENTION

As a result of diligent investigation aimed at solving the above problems, the inventors arrived at the present invention upon discovering that an enteric coating having an elution control function can be produced by employing a coating that comprises a mixture of an enteric polymer and a water-insoluble polymer.

Specifically, the above object is achieved by a preparation composition comprising (1) a core containing an acid-unstable physiologically active compound, and (2) a coating that covers the core, the coating comprising at least one layer and containing a water-insoluble polymer and an enteric polymer.

The coating of the above-mentioned preparation composition involves at least one layer and combines an elution control function with an enteric function, and also contributes to higher productivity.

In a preferred aspect of the present invention, the preparation composition is characterized in that the water-insoluble polymer is contained in an amount of 20 to 80 wt% based on a total weight of the water-insoluble polymer and the enteric polymer contained in the coating.

In a preferred aspect of the present invention, the preparation composition is characterized in that the coating further contains a plasticizer.

In a preferred aspect of the present invention, the preparation composition is characterized in that the core further contains an alkaline substance.

In a preferred aspect of the present invention, the preparation composition is characterized in that the water-insoluble polymer is selected from the group consisting of ethyl cellulose, aminoalkyl methacrylate copolymer, and shellac.

In a preferred aspect of the present invention, the preparation composition is characterized in that the enteric polymer is selected from the group consisting of hydroxypropyl methyl cellulose acetate succinate, hydroxypropyl methyl cellulose phthalate, methacrylic acid-methyl methacrylate copolymer, and methacrylic acid-ethyl acrylate copolymer.

In a preferred aspect of the present invention, the preparation composition is characterized in that the plasticizer is selected from the group consisting of triethyl citrate, cetyl alcohol, glycerol fatty acid ester, propylene glycol, polyethylene glycol, hydrogenated castor oil, hydrogenated rapeseed oil, and silicone oil.

In a preferred aspect of the present invention, the preparation composition is characterized in that the alkaline substance is selected from the group consisting of sodium hydroxide, potassium hydroxide, sodium carbonate, and potassium carbonate.

In a preferred aspect of the present invention, the preparation composition is characterized in that the physiologically active compound is a benzimidazole-based compound or a physiologically acceptable salt thereof.

In a preferred aspect of the present invention, the preparation composition is characterized in that the benzimidazole-based compound is rabeprazole, and the physiologically acceptable salt thereof is a sodium salt.

The above object is also achieved by a process for producing a preparation composition, comprising spraying a solution containing a mixture of a water-insoluble polymer and an enteric polymer onto a core containing an acid-unstable physiologically active compound so as to form a coating that covers the core.

In a preferred aspect of the present invention, the process is characterized in that the water-insoluble polymer is contained in an amount of 20 to 80 wt% based on a total weight of the water-insoluble polymer and the enteric polymer contained in the coating.

In a preferred aspect of the present invention, the process is characterized in that the coating further contains a plasticizer.

In a preferred aspect of the present invention, the process is characterized in that the core further contains an alkaline substance.

In a preferred aspect of the present invention, the process is characterized in that the water-insoluble polymer is selected from the group consisting of ethyl cellulose, aminoalkyl methacrylate copolymer, and shellac.

In a preferred aspect of the present invention, the process is characterized in that the enteric polymer is selected from the group consisting of hydroxypropyl methyl cellulose acetate succinate, hydroxypropyl methyl cellulose phthalate, methacrylic acid-methyl methacrylate copolymer, and methacrylic acid-ethyl acrylate copolymer.

In a preferred aspect of the present invention, the process is characterized in that the plasticizer is selected from the group consisting of triethyl citrate, cetyl alcohol, glycerol fatty acid ester, propylene glycol, polyethylene glycol, hydrogenated castor oil, hydrogenated rapeseed oil, and silicone oil.

In a preferred aspect of the present invention, the process is characterized in that the alkaline substance is selected from the group consisting of sodium hydroxide, potassium hydroxide, sodium carbonate, and potassium carbonate.

In a preferred aspect of the present invention, the process is characterized in that the physiologically active compound is a benzimidazole-based compound or a physiologically acceptable salt thereof.

In a preferred aspect of the present invention, the process is characterized in that the benzimidazole-based compound is rabeprazole, and the physiologically acceptable salt thereof is a sodium salt.

The term "acid-unstable" used in the present invention means chemically unstable in gastric acid and/or an aqueous solution with an acidic pH, and that decomposition is promoted.

The term "core" used in the present invention refers to a core substance that contains just a physiologically active compound, or also contains various preparation additives normally used in the field of this technology, and refers to a substance in the form of a tablet, a granule, a fine granule, and the like.

Further, the term "preparation composition" used in the present invention means a preparation composition comprising a coated core, or a preparation composition in which the above-mentioned core is covered with an intermediate coating, which is then covered.

### BRIEF DESCRIPTION OF DRAWINGS

Fig. 1 is a graph showing the dissolution behavior of the preparation composition according to the present invention;
Fig. 2 is a graph showing the dissolution behavior of another preparation composition according to the present invention;
Fig. 3 is a graph showing the dissolution behavior of yet another preparation composition according to the present invention;
Fig. 4 is a graph showing the dissolution behavior of the preparation composition according to the present invention when a coverage amount was substantially constant (19.3 ± 0.7 mg); and
Fig. 5 is a graph showing the stability of the preparation composition according to the present invention in an acidic solution.

### BEST MODE FOR CARRYING OUT THE INVENTION

Preferred embodiments of the present invention will now be described in detail.

The present invention provides a preparation composition, comprising a core containing an acid-unstable physiologically active compound, and a coating that covers this core, wherein the above-mentioned compound is unstable in acids, and the coating includes a mixture of a water-insoluble polymer and an enteric polymer.

The preparation composition according to the present invention is preferably a solid preparation composition, and can be formulated into any form that is ordinarily administered orally to humans, such as a tablet, a granule, a fine granule, a capsule, and the like.

There are no particular restrictions on the acid-unstable physiologically active compound contained in the core used in the present invention, but examples of such compound include a gastric acid secretion inhibitor and an antibiotic and the like. The above-mentioned gastric acid secretion inhibitor includes benzimidazole-based compound and a physiologically acceptable salt thereof, for example, while the above-mentioned antibiotic includes penicillin-based antibiotics and macrolide-based antibiotics, and the like.

Specific examples of the above-mentioned benzimidazole compound include rabeprazole, omeprazole, pantoprazole, and lansoprazole. Examples of physiologically acceptable salts thereof include alkali metal salt and alkaline earth metal salt. Specific examples of alkali metal salt include sodium salt and potassium salt, while alkaline earth metal salt include magnesium salt and the like. The following are examples of the structural formulas of compounds suitable for in the present invention.

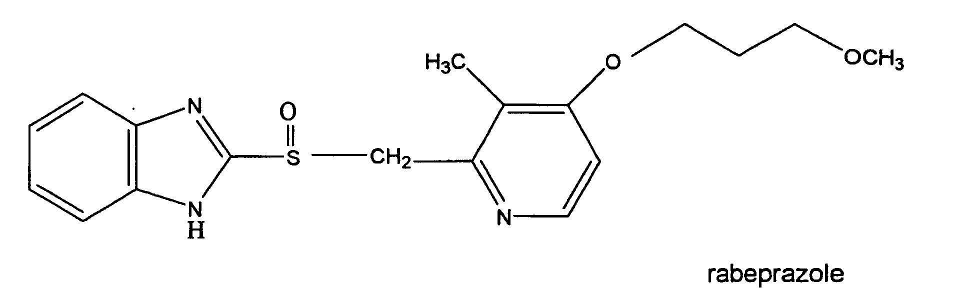

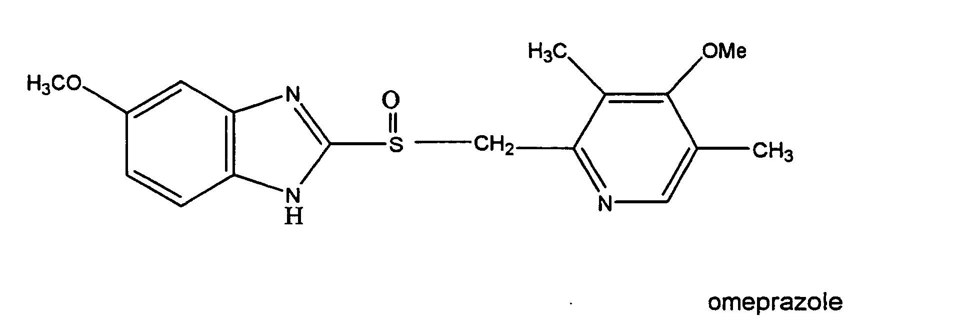

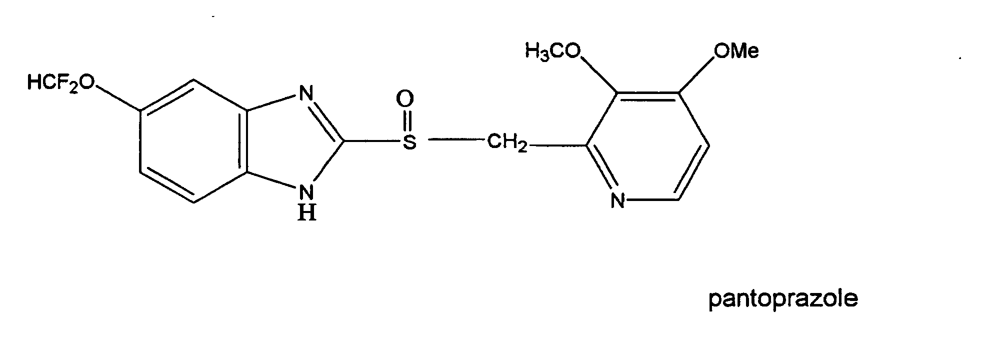

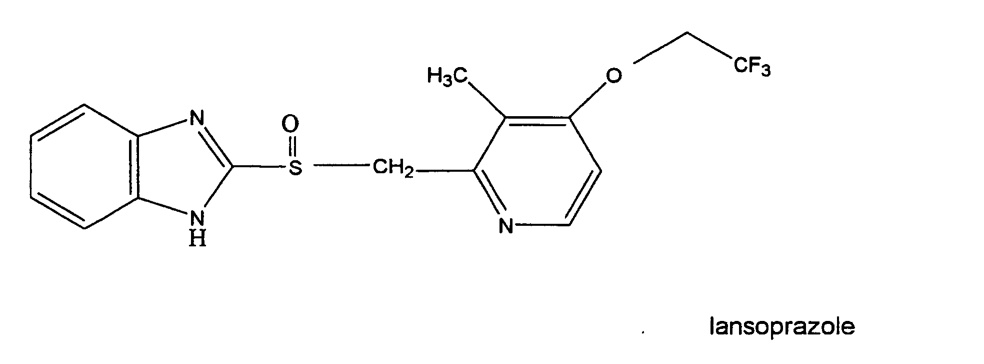

The benzimidazole-based compound used in the present invention can be produced by a known method. For instance, it can be produced by one of the methods disclosed in Japanese Laid-Open Patent Applications S52-62275, S54-141783, and H1-6270.

It is particularly favorable in the present invention if the benzimidazole-based compound is rabeprazole, and the physiologically acceptable salt thereof is a sodium salt.

A specific example of the above-mentioned penicillin-based antibiotics is benzyl penicillin, and specific examples of the above-mentioned macrolide-based antibiotics include erythromycin and clarithromycin.

The composition forming the core used in the present invention (hereinafter referred to as "core composition") preferably is a composition containing an acid-unstable physiologically active compound such as a benzimidazole-based compound, and contains any of various other preparation additives. There are no particular restrictions on the preparation additives but examples of such additives include an excipient, a stabilizer, a disintegrant, a binder, a lubricant and the like.

The above-mentioned benzimidazole-based compounds are known to be extremely unstable in an acidic state, and in core compositions and/or preparation compositions in which ordinary additives have been mixed, these compounds are extremely unstable when stored under conditions of elevated temperature and humidity, which promotes decomposition. During this decomposition, not only is there an increase in impurities, but it has also been observed that core compositions and/or preparation composition containing a benzimidazole-based compound undergo a particularly pronounced color change.

An alkaline substance is preferably added to the core as a stabilizer. Specific examples of this alkaline substance include sodium hydroxide, potassium hydroxide, sodium carbonate, potassium carbonate and the like. These substances can be used singly or in combinations or two or more types.

The proportion in which the benzimidazole-based compound and the alkaline substance are mixed is preferably 0.001 to 2 weight parts, and more preferably 0.005 to 1 weight part, and even more preferably 0.01 to 0.5 weight part of alkaline substance per 1 weight part of benzimidazole-based compound.

The above method of stabilizing a benzimidazole-based compound with the alkaline substance used in the present invention has a noticeably favorable effect in that it not only increases the stability of the benzimidazole-based compound itself, but also limits color changes.

Any excipient that is ordinarily used, such as lactose or mannitol, can be used to produce the core containing a benzimidazole-based compound used in the present invention. There are no particular restrictions on the binder used in the present invention, but hydroxypropyl cellulose is preferable, and crospovidone is preferable as the disintegrant.

It is known that the crospovidone commonly used as the disintegrant can be pulverized to reduce the disintegrating power and wetting power ordinarily associated with the disintegrant. Crospovidone that has been pulverized and/or seived to a small particle size is used as the stabilizer of the benzimidazole-based compound in the present invention, and can be added in an amount over the amount in which it is usually added as the disintegrant (no more than 10%). The average particle size of this pulverized and/or seived crospovidone (hereinafter referred to as "fine crospovidone") is preferably from a few microns to 50 µm.

Therefore, if crospovidone or fine crospovidone with an average particle size of from a few microns to 50 µm is used in the core composition or preparation composition according to the present invention, it will contribute, together with the above-mentioned alkaline substance, to better stabilization of the benzimidazole-based compound. Of course, fine crospovidone and ordinary crospovidone may also be used together.

The proportion in which the crospovidone and the benzimidazole-based compound are mixed in the present invention is preferably 0.1 to 10 weight parts, and more preferably 0.2 to 8 weight parts, and even more preferably 0.5 to 5 weight parts of crospovidone per 1 weight part of benzimidazole-based compound.

It should be noted that crospovidone frequently contains a trace amount of peroxide as an impurity, although this varies with the manufacturer and lot. Because the benzimidazole-based compound has the property of being readily oxidized, an antioxidant may be added at the time of the crospovidone addition. Specific examples of antioxidants include sodium sulfite, sodium pyrosulfite, vitamin E, rongalite, thioglycerol, sodium thiosulfate, salt of ascorbic acid, acetylcysteine, and the like, but the antioxidant is not limited to the above.

The core according to the present invention can be produced by any commonly used method. For example, crospovidone, sodium hydroxide and/or potassium hydroxide, or the like is mixed as a stabilizer into a benzimidazole-based compound, the excipient or the binder is added, and wet or dry granulation is performed. If needed, crospovidone (used as the disintegrant) or the lubricant can be added and compression-molded is performed to produce tablets. Of course, the process for producing a core composition used in the present invention is not limited to the above method.

Specific examples of the above-mentioned lubricant include magnesium stearate, sodium stearyl fumarate, calcium stearate, and stearic acid, as well as hydrogenated oils and other lipids. Sodium stearyl fumarate, calcium stearate, and stearic acid are preferable, and sodium stearyl fumarate is particularly favorable.

There are no particular restrictions on the proportion in which the sodium stearyl fumarate or other lubricant is added in the present invention, but the amount is preferably 0.01 to 20%, and more preferably 0.05 to 15%, and even more preferably 0.1 to 10%, with respect to the uncovered core containing a benzimidazole-based compound or an alkali metal salt thereof.

The above-mentioned lubricant preferably has an average particle size of 0.5 to 50 µm, and more preferably 1 to 50 µm.

The coating that covers the above-mentioned core will now be described. The coating according to the present invention is an enteric coating that contains a water-insoluble polymer and an enteric polymer. Because an enteric polymer generally exhibits acidity, it preferably does not come into direct contact with the benzimidazole-based compound, which is unstable to acids.

In view of this, it is preferable to provide an inert intermediate coating between the core containing the benzimidazole-based compound and the above-mentioned enteric coating for the preparation composition according to the present invention

The term "inert" as used above means having no adverse effect on the stability of the benzimidazole-based compound.

There are no particular restrictions on the inert intermediate coating, which may be a coating containing a water-soluble polymer, a water-soluble or water-dispersible substance, or a water-insoluble substance, or may be a coating containing two or more types of these. Specific examples of the substance forming the intermediate coating include hydroxypropyl cellulose, hydroxypropyl methyl cellulose, aminoalkyl methacrylate copolymer E, lactose, mannitol, starch, crystalline cellulose, ethyl cellulose, vinyl acetate and the like.

As disclosed in Japanese Laid-Open Patent Application H1-29062, when an intermediate coating comprising a water-insoluble substance is provided, microparticles that are insoluble in water can also be mixed into the intermediate coating.

The coating accroding to the present invention is characterized by comprising at least one layer and containing a mixture of a water-insoluble polymer and an enteric polymer. The coating according to the present invention may also comprise two or more layers of coating with different mix ratios between the water-insoluble polymer and the enteric polymer, or may comprise a gradation coating in which the mix ratio between the water-insoluble polymer and the enteric polymer varies.

The desired sustained release can be imparted with the present invention by suitably selecting the types of water-insoluble polymer and enteric polymer, the blend ratio of these polymers, and other such variables. Imparting this sustained release property makes it possible to control the rate of dissolution of the physiologically active compound that is the active ingredient.

There are no particular restrictions on the water-insoluble polymer used in the present invention as long as it substantially does not dissolve in water and has the property of dissolving or uniformly dispersing in methanol, ethanol, propanol, isopropanol, acetone, or another such organic solvent, but specific examples of the water-insoluble polymer include ethyl cellulose, aminoalkyl methacrylate copolymer, shellac and the like. These can be used singly in the present invention, or two or more types can be combined.

There are no particular restrictions on the enteric polymer used in the present invention, but specific examples of the enteric polymer include hydroxypropyl methyl cellulose phthalate (trade names HP-55 and HP-60, made by Shin-Etsu Chemical Industries), methacrylic acid-methyl methacrylate copolymer (trade names Eudragit L100, Eudragit L100-55, and Eudragit S100, made by Roehm Pharma), methacrylic acid-ethyl acrylate copolymer (trade name Eudragit L30D-55, made by Roehm Pharma), and hydroxypropyl acetate succinate (Acoat, made by Shin-Etsu Chemical Industries). These can be used singly or in combinations of two or more types. Of these, it is preferable to use a methacrylic acid-methyl methacrylate copolymer (trade name Eudragit L100 or L100-55, also known as methacrylic acid copolymer L), Eudragit S100 (also known as methacrylic acid copolymer S, made by Roehm Pharma), or a mixture of these.

In a preferred aspect of the present invention, the blend ratio between the water-insoluble polymer and the enteric polymer can be suitably selected from a range in which it is possible to control the dissolution of the active ingredient, and can be varied as dictated by the physiologically active compound, the sustained release type, the release rate, and the like.

In particular, to maintain the effective blood concentration of the benzimidazole-based compound for a long time, it is preferable for the water-insoluble polymer to be added in an amount of 15 to 85 wt%, and more preferably 20 to 80 wt%, and even more preferably 25 to 75 wt%, with respect to the total weight of the water-insoluble polymer and the enteric polymer in the composition forming the coating. It will be difficult to maintain the effective blood concentration over an extended period if the water-insoluble polymer content is less than 20 wt%.

Various additives may be added to the coating containing a mixture of the above-mentioned polymers and used to cover the core according to the present invention. Examples of such additives include a plasticizer, a colorant, and a lubricant.

Specific examples of the plasticizer used in the present invention include triethyl citrate, cetyl alcohol, glycerol fatty acid ester, propylene glycol, polyethylene glycol, hydrogenated castor oil, hydrogenated rapeseed oil, silicone oil and the like.

Specific examples of the colorant used in the present invention include a food dye, a lake dye, caramel, carotene, annatto (a natural dye derived from the annatto tree), cochineal, iron oxide, titanium dioxide, and the like, as well as a opaque colorant whose main components are a lake dye and syrup. More specific examples include food aluminum lake dye, such as a food red No. 2 and No. 3, yellow No. 4 and No. 5, green No. 3, blue No. 1 and No. 2, violet No. 1, and carmine (carminic acid aluminum salt), pearl essence (whose main component is guanine), and the like.

Specific examples of the lubricant used in the present invention include magnesium stearate, talc, synthetic magnesium silicate, and microparticulate silicon oxide.

The amounts in which these additives are added, and when they are added, do not matter with the present invention as long as they are within the normal ranges employed in the field of pharmaceutical technology.

As to the method by which the core according to the present invention is covered with the coating containing the water-insoluble polymer and the enteric polymer, an enteric and sustained-release coating solution prepared by dissolving or dispersing the water-insoluble polymer and the enteric polymer can be applied by pan coating, fluidized bed coating, agitation fluidized bed coating, ant the like. Here, the core or a core that has been covered with an inert intermediate coating is agitated and/or fluidized, and into this bed is sprayed the enteric, sustained-release coating solution produced by dissolving or suspending the water-insoluble polymer and the enteric polymer in a solvent, the solvent is then removed by drying with hot forced air, and at least one layer of coating is formed on the outer layer of the core.

A solution in which the water-insoluble polymer and the enteric polymer have already been adjusted to a specific blend ratio may be applied, or a solution of the water-insoluble polymer and a solution of the enteric polymer may be prepared separately, and a gradation coating formed by varying the mix ratio of the two solutions over time with a pump or the like. It is also possible to laminate numerous layers of coating with the same or different blend ratios of the water-insoluble polymer and the enteric polymer.

There are no particular restrictions on the solvent used for the above-mentioned coating solution as long as the water-insoluble polymer and the enteric polymer can both be dissolved or suspended therein, but examples of the solvent include alcohols, such as methanol, ethanol, n-propanol, isopropanol, n-butanol, 2-methoxyethanol, 2-ethoxyethanol; hydrocarbons, such as hexane, cyclohexane, petroleum ether, petroleum benzine, ligroin, benzene, toluene, xylene; ketones, such as acetone, methyl ethyl ketone; halogenated hydrocarbons, such as dichloromethane, chloroform, carbon tetrachloride, ethylene dichloride, trichloroethylene, 1,1,1-trichloroethane; esters, such as acetic acid methyl ester, acetic acid ethyl ester, acetic acid butyl ester; and ethers, such as isopropyl ether, dioxane. These solvents should be selected according to the types of the water-insoluble polymer and the enteric polymer being used, and two or more types may be combined as needed.

Of these, an alcohol is a more favorable solvent, and ethanol is even more preferable solvent.

As discussed above, the preparation composition according to the present invention comprises a core covered with a coating containing the water-insoluble polymer and the enteric polymer, but this preparation composition can be further covered with a moisture-proof coating. The term "moisture-proof coating" as used here means a coating that suppresses the permeation of water vapor, and in terms of function, includes a coating in which the coating itself suppresses the permeation of water vapor, and a coating in which water vapor is trapped in the coating so that the water vapor cannot penetrate to the interior.

This moisture-proof coating has the function of improving stability by preventing the infiltration of moisture into the benzimidazole-based compound, and of preventing the cracking and deformation of the core composition that originate in swelling during the absorption of moisture by finely pulverized crospovidone.

This moisture-proof coating may be either water-soluble or water-insoluble, and examples of such moisture-proof coating include coatings composed of polyvinyl acetal diethylaminoacetate, HA Sankyo (a mixture of fumaric acid, stearic acid, hydroxypropyl methyl cellulose, and polyvinyl acetal diethylaminoacetate), polyvinyl alcohol, and the like, coatings produced by mixing one or more of cellulose, such as hydroxypropyl cellulose, hydroxypropyl methyl cellulose, and other cellulose derivatives, and/or sugar coatings whose main component is sucrose, and sugar coatings whose main component is maltitol, although the moisture-proof coating is not limited to the above.

The present invention will now be described in more specific terms through examples, but the present invention is not limited in any way by way of these examples.

### Example 1

6.4 kg of mannitol, 2.25 kg of crospovidone, and 0.2 kg of hydroxypropyl cellulose were added to and mixed with 1.5 kg of a sodium salt of rabeprazole, and the mixture was added to 0.1 kg of sodium hydroxide dissolved in 3.75 kg of ethanol, and granulated. The granules thus produced were dried for 12 hours at 50°C, and then passed through a 1.5 mm screen, after which 0.6 kg of crospovidone and 0.15 kg of sodium stearyl fumarate (made by Penwest) were added and mixed, and the mixture was tableted in a rotary compression machine. The tablets thus obtained each weighed 150 mg. 3 kg of tablets were put into a coating pan and coated with an intermediate coating of the following composition, in an amount of 5.7 mg per tablet.

| | |
|---|---|
| hydroxypropyl cellulose | 500 g |
| magnesium stearate | 125 g |
| ethanol | 10,000 g |

Next, 200 g of the tablets covered with an intermediate coating were given an enteric, sustained-release coating of the following composition, in an amount of 9.8 mg, with a pan coating machine.

| | |
|---|---|
| Eudragit L100 | 40 g |
| ethyl cellulose | 40 g |
| talc | 7.5 g |
| titanium dioxide | 4.5 g |
| cetyl alcohol | 8 g |
| ethanol | 1250 g |

### Example 2

200 g of the tablets covered with an intermediate coating obtained in Example 1 were sprayed with an enteric, sustained-release coating solution, in an amount of 14.2 mg, with a pan coating machine in the same manner as in Example 1.

### Example 3

200 g of the tablets covered with an intermediate coating obtained in Example 1 were sprayed with an enteric, sustained-release coating solution, in an amount of 18.7 mg, with a pan coating machine in the same manner as in Example 1.

### Example 4

200 g of the tablets covered with an intermediate coating obtained in Example 1 were sprayed with an enteric, sustained-release coating solution, in an amount of 31.5 mg, with a pan coating machine in the same manner as in Example 1.

### Example 5

200 g of the tablets covered with an intermediate coating obtained in Example 1 were sprayed with the following enteric, sustained-release coating solution, in an amount of 8.5 mg, with the same pan coating machine as in Example 1.

| | |
|---|---|
| Eudragit L100 | 60 g |
| ethyl cellulose | 20 g |
| talc | 7.5 g |
| titanium dioxide | 4.5 g |
| cetyl alcohol | 8 g |
| ethanol | 1250 g |

### Example 6

200 g of the tablets covered with an intermediate coating obtained in Example 1 were sprayed with the same enteric, sustained-release coating solution as in Example 5, in an amount of 13.7 mg, with a pan coating machine in the same manner as in Example 1.

### Example 7

200 g of the tablets covered with an intermediate coating obtained in Example 1 were sprayed with the same enteric, sustained-release coating solution as in Example 5, in an amount of 19.2 mg, with a pan coating machine in the same manner as in Example 1.

### Example 8

200 g of the tablets covered with an intermediate coating obtained in Example 1 were sprayed with the same enteric, sustained-release coating solution as in Example 5, in an amount of 25.1 mg, with a pan coating machine in the same manner as in Example 1.

### Example 9

200 g of the tablets covered with an intermediate coating obtained in Example 1 were sprayed with the same enteric, sustained-release coating solution,as in Example 5, in an amount of 31.1 mg, with a pan coating machine in the same manner as in Example 1.

### Example 10

200 g of the tablets covered with an intermediate coating obtained in Example 1 were sprayed with the following enteric, sustained-release coating solution, in an amount of 4.6 mg, with a pan coating machine in the same manner as in Example 1.

| | |
|---|---|
| Eudragit L100 | 20 g |
| ethyl cellulose | 60 g |
| talc | 7.5 g |
| titanium dioxide | 4.5 g |
| cetyl alcohol | 8 g |
| ethanol | 1250 g |

### Example 11

200 g of the tablets covered with an intermediate coating obtained in Example 1 were sprayed with the same enteric, sustained-release coating solution as in Example 10, in an amount of 9.4 mg, with a pan coating machine in the same manner as in Example 1.

### Example 12

200 g of the tablets covered with an intermediate coating obtained in Example 1 were sprayed with the same enteric, sustained-release coating solution as in Example 10, in an amount of 14.8 mg, with a pan coating machine in the same manner as in Example 1.

### Example 13

200 g of the tablets covered with an intermediate coating obtained in Example 1 were sprayed with the same enteric, sustained-release coating solution as in Example 10, in an amount of 20.0 mg, with a pan coating machine in the same manner as in Example 1.

### Example 14

200 g of the tablets covered with an intermediate coating obtained in Example 1 were sprayed with the same enteric, sustained-release coating solution as in Example 10, in an amount of 27.7 mg, with a pan coating machine in the same manner as in Example 1.

### Control 1

200 g of the tablets covered with an intermediate coating obtained in Example 1 were sprayed with the following enteric coating solution, in an amount of 20.0 mg, with a pan coating machine in the same manner as in Example 1.

| | |
|---|---|
| Eudragit L100 | 80 g |
| Talc | 7.5 g |
| titanium dioxide | 4.5 g |
| cetyl alcohol | 8 g |
| ethanol | 1250 g |

The tablets (preparation compositions) obtained in Examples 1 to 14 and Control 1 were evaluated by the following methods.

### Evaluation Method 1

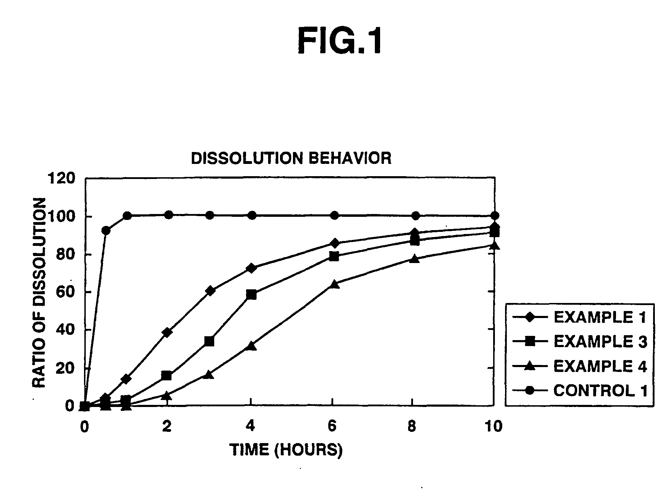

The tablets obtained in Examples 1, 3 to 7, and 9 to 14, and Control 1 were subjected to a dissolution test by paddle method in 1000 mL of a 0.1 M Na₂CO₃ aqueous solution (solution temperature: 37°C) that had been adjusted to a pH of 11. The dissolution was measured by measuring the rabeprazole absorbance of the sampled solution by UV method. These results are given in Figs. 1 to 4.

It can be seen from Figs. 1 to 3 that dissolution occurred quickly with Control 1, which featured tablets covered with a Eudragit L100 coating (an enteric polymer) that did not contain a water-insoluble polymer. On the other hand, it was found that the rabeprazole, which is the physiologically active compound, eluted gradually in the various Examples.

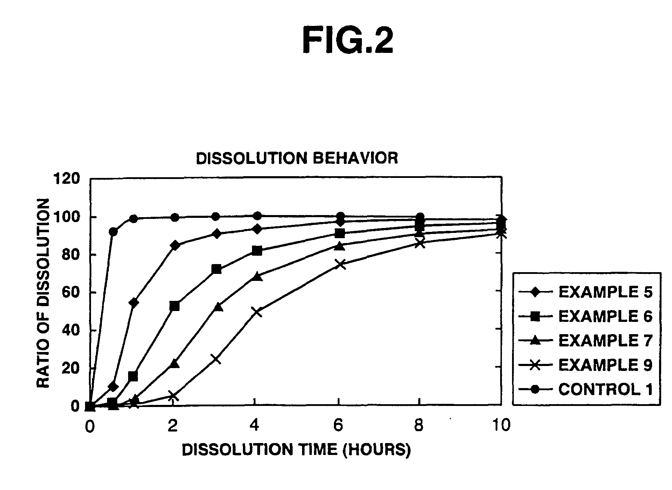

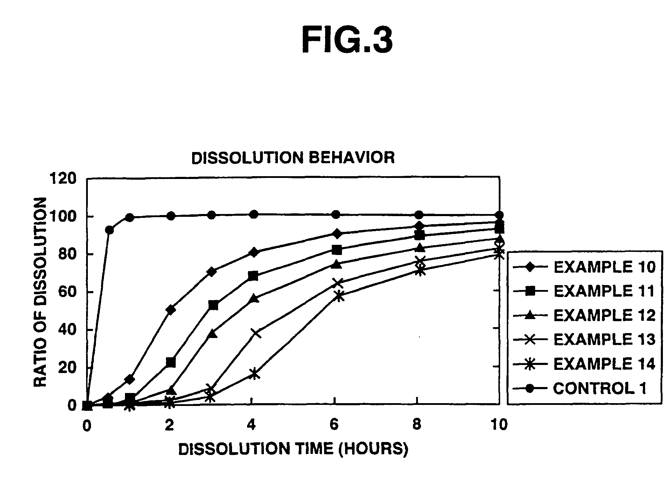

In Examples 1, 3, and 4 shown in Fig. 1, the compositions of the coatings are the same, but the coverage amounts of the coatings are different, so the dissolution rate varies with the coverage amount, with dissolution slowing down as the coverage amount increases, and it was confirmed that the dissolution rate can be controlled by varying the coverage amount of the coating. Similar results were obtained for Figs. 2 and 3 as well.

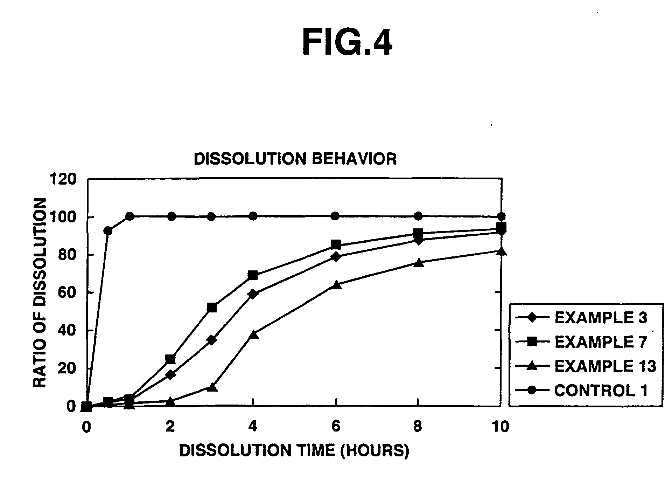

Fig. 4 is a graph showing the dissolution behavior of the preparation compositions prepared in Examples 3, 7, and 13 and Control 1 when the coverage amount of the coating according to the present invention was 19.3 ± 0.7 mg.

As is clear from Fig. 4, when the coverage amount is substantially the same, the higher is the proportion in which the water-insoluble polymer is contained versus the total weight of the water-insoluble polymer and the enteric polymer in the coating, the more the dissolution rate decreases, which proves that the dissolution rate can be controlled by varying the blend proportions of the two polymers.

### Evaluation Method 2

The stability of the tablets obtained in the Examples and the Control in an acidic solution was evaluated as described below.

There are two ways to evaluate dissolution under acidic conditions: acid resistant weight increase and acid resistance. With acid resistant weight increase, a tablet is weighed at the outset and left for 2 hours in a test solution (37°C solution temperature) with a pH of 1.0, and then weighed again to measure the weight increase.

With acid resistance, a tablet left for 2 hours in a test solution (37°C solution temperature) with a pH of 1.0, and then the rabeprazole (active ingredient) content is measured, and the percentage remaining versus the content in the tablet prior to immersion in the test solution is calculated.

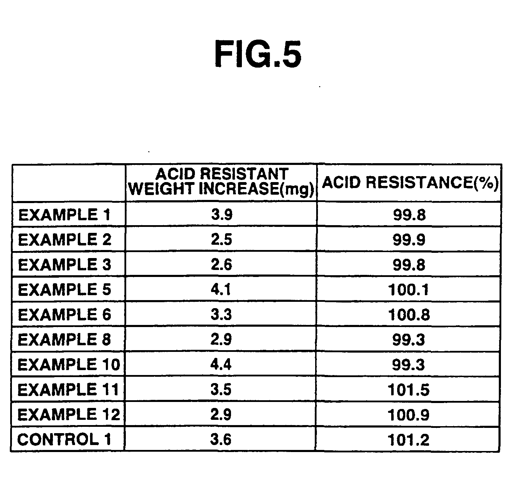

Fig. 5 shows the results for acid resistant weight increase and acid resistance in the various examples. The coating used in Control 1 was a conventional enteric coating, and a comparison of the results for this coating with those of the various Examples reveals that the rabeprazole was stable under acidic conditions in all of the Examples, and the coatings used in the various Examples had the function of an enteric coating.

### INDUSTRIAL APPLICABILITY

As described above, with the preparation composition according to the present invention, which is covered by a coating composed of a mixture of a water-insoluble polymer and an enteric polymer, and with the process for producing the same, it is possible to control the sustained release of an active ingredient covered by this coating by means of a simple process. Thus imparting the desired sustained release properties to an active ingredient affords an improvement in therapeutic efficacy in which the effective blood concentration in an organism is maintained over an extended period of time.

## Claims

1. A preparation composition, comprising:
(1) a core containing an acid-unstable physiologically active compound; and
(2) a coating that covers the core, the coating comprising at least one layer and containing a water-insoluble polymer and an enteric polymer.

2. The preparation composition according to Claim 1, wherein the water-insoluble polymer is contained in an amount of 20 to 80 wt% based on a total weight of the water-insoluble polymer and the enteric polymer contained in the coating.

3. The preparation composition according to Claim 1, wherein the coating further comprises a plasticizer.

4. The preparation composition according to Claim 1, wherein the core further comprises an alkaline substance.

5. The preparation composition according to Claim 1, wherein the water-insoluble polymer is selected from the group consisting of ethyl cellulose, aminoalkyl methacrylate copolymer, and shellac.

6. The preparation composition according to Claim 1, wherein the enteric polymer is selected from the group consisting of hydroxypropyl methyl cellulose acetate succinate, hydroxypropyl methyl cellulose phthalate, methacrylic acid-methyl methacrylate copolymer, and methacrylic acid-ethyl acrylate copolymer.

7. The preparation composition according to Claim 3, wherein the plasticizer is selected from the group consisting of triethyl citrate, cetyl alcohol, glycerol fatty acid ester, propylene glycol, polyethylene glycol, hydrogenated castor oil, hydrogenated rapeseed oil, and silicone oil.

8. The preparation composition according to Claim 4, wherein the alkaline substance is selected from the group consisting of sodium hydroxide, potassium hydroxide, sodium carbonate, and potassium carbonate.

9. The preparation composition according to Claim 1, wherein the physiologically active compound is a benzimidazole-based compound or a physiologically acceptable salt thereof.

10. The preparation composition according to Claim 9, wherein the benzimidazole-based compound is rabeprazole, and the physiologically acceptable salt thereof is a sodium salt.

11. A process for producing a preparation composition, comprising:
spraying a solution containing a mixture of a water-insoluble polymer and an enteric polymer onto a core containing an acid-unstable physiologically active compound so as to form a coating that covers the core.

12. The process according to Claim 11, wherein the water-insoluble polymer is contained in an amount of 20 to 80 wt% based on a total weight of the water-insoluble polymer and the enteric polymer contained in the coating.

13. The process according to Claim 11, wherein the coating further comprises a plasticizer.

14. The process according to Claim 11, wherein the core further comprises an alkaline substance.

15. The process according to Claim 11, wherein the water-insoluble polymer is selected from the group consisting of ethyl cellulose, aminoalkyl methacrylate copolymer, and shellac.

16. The process according to Claim 11, wherein the enteric polymer is selected from the group consisting of hydroxypropyl methyl cellulose acetate succinate, hydroxypropyl methyl cellulose phthalate, methacrylic acid-methyl methacrylate copolymer, and methacrylic acid-ethyl acrylate copolymer.

17. The process according to Claim 13, wherein the plasticizer is selected from the group consisting of triethyl citrate, cetyl alcohol, glycerol fatty acid ester, propylene glycol, polyethylene glycol, hydrogenated castor oil, hydrogenated rapeseed oil, and silicone oil.

18. The process according to Claim 14, wherein the alkaline substance is selected from the group consisting of sodium hydroxide, potassium hydroxide, sodium carbonate, and potassium carbonate.

19. The process according to Claim 11, wherein the physiologically active compound is a benzimidazole-based compound or a physiologically acceptable salt thereof.

20. The process according to Claim 19, wherein the benzimidazole-based compound is rabeprazole, and the physiologically acceptable salt thereof is a sodium salt.
